# EUROPEAN PATENT SPECIFICATION

(11) **EP 0 535 682 B1**
(45) Date of publication and mention of the grant of the patent: **12.07.1995**
(21) Application number: 92116848.0
(22) Date of filing: 01.10.1992
(51) Int. Cl.: C07C 15/46, C07B 35/06, C07C 1/30

(54) **Preparation of aryl-substituted olefins**
Herstellung von aryl-substituierten Olefinen
Préparation d'oléfines aryliques

(30) Priority: 02.10.1991 US 769718
(43) Date of publication of application: 07.04.1993
(73) Proprietor: ALBEMARLE CORPORATION, Richmond, Virginia 23219 (US)
(72) Inventor: Laurenzo, Kathleen Susan, Baton Rouge, Louisiana 70817 (US); Stahly, Glenn Patrick, Baton Rouge, Louisiana 70810 (US); Orihuela, Felix Manuel, Baton Rouge, Louisiana 70816 (US)
(74) Representative: Sandmair, Kurt, Dr. Dr.

(56) References cited:
- FR-A- 2 328 689
- US-A- 4 543 433
- BULLETIN OF THE CHEMICAL SOCIETY OF JAPAN vol. 54, no. 4, April 1981, TOKYO JP pages 1265 - 1266 T. OKAMOTO ET AL 'THE TRANSITION-METAL CATALYZED DEHALOGENATION OF AROMATIC HALIDES BY NaOH-ALCOHOLS. A FACILE METHOD OF DESTROYING AROMATIC POLYHALIDES'
- TETRAHEDRON LETTERS. no. 24, 1978, OXFORD GB pages 2075 - 2078 J. TSUJI ET AL 'FORMATION OF A TERMINAL CONJUGATED DIENE SYSTEM BY PALLADIUM CATALYZED ELIMINATION REACTIONS OF ALLYLIC ACETATES AND PHENYL ETHERS'

## Description

The present invention relates to a process for the preparation of aromatic-substituted olefins by catalytically dehydrohalogenating an aryl-substituted alkyl chloride.

The intramolecular removal of a hydride to form an olefin is an important reaction for metal alkyl decomposition and for metal catalyzed olefin isomerizations. It has traditionally been assumed that the presence or absence of β-hydrogens is the most important single factor determining whether an alkyl ligand is stable and will yield the desired olefin. Accordingly, a great deal of research has been carried out with ligands such as methyl, benzyl, neopentyl, (Me₃CCH₂-) and trimethylbutyl. However, these elimination reactions need ligand dissociation and a vacant coordination site on the metal before they can occur. As such, alkyl complexes may be stable if they are sufficiently substitution-inert, e.g., [(H₂O)₅CHCH(CH₃)₂]²⁺. Furthermore, because of the instability of the olefins that would be formed by β-elimination, 1-adamantyl and 1-norbornyl form stable (isolable) complexes with a number of transition metals.

While palladium is generally recognized as a catalytic metal most useful in effecting the reductions of organic compounds, special cases have been discovered where the reverse reaction has been observed using this material. Thus, for example, aromatization of six-membered alicyclic rings has been shown to occur in the presence of palladium metal. See, for example, Rylander, "Organic Synthesis with Noble Metal Catalysts", pp. 1-59, Academic Press, New York, 1953.

U. S. Patent No, 4,543,433 discloses the preparation of a 2-methyl-6-methylene-1,3,7-octatriene by a route comprising the dehydrochlorination of 3-chloro-2-methyl-6-methylene-1,7-octadiene in the presence of a palladium catalyst, a ligand. The latter is described as a phosphine, diphosphine, phosphite, arsine or stilbene. The reaction, carried out with an inorganic base, produces the highly conjugated product at high conversions and in good yield. Similar reactions have been carried out by the palladium catalyzed elimination reactions of allylic acetates and phenyl ethers. [Tsuji, et al., Tetrahedron Letters, 24, 2075-2078 (1978)].

Okamoto und Oka [Bull. Chem. Soc. Japan, 54, 1265-1266 (1981)] teach to use rhodium(III)chloride or palladium(II)acetate and triphenylphosphine for dehalogenation of aromatic halides by heating with NaOH in alcoholic solvents. When dehalogenating (1-chloroethyl)-benzene equal amounts of ethyl benzene and styrene were obtained.

Palladium compounds are also used for carbonylation reactions as it is for example disclosed in FR-A-2 328 689. This disclosure pertains to the carbonylation of α-arylvinyl compounds using a palladium catalyst. The vinyl compounds are prepared by dehalogenation of an α- or β-haloethylaryl compound using Al or alkali, but not Pd.

It is an object of the present invention to carry out a palladium-catalyzed dehydrohalogenation of saturated alkyl halides.

According to the present invention, compounds of the formula 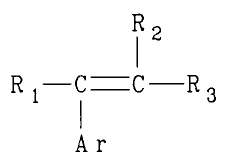
where Ar is phenyl, substituted phenyl, naphthyl, substituted naphthyl, heteroaryl or substituted heteroaryl; R₁, R₂ and R₃ are individually the same or different and are hydrogen, alkyl, cycloalkyl, alkoxyalkyl, alkylthioalkyl, phenylalkyl, substituted phenylalkyl, heteroarylalkyl, substituted heteroarylalkyl or cycloalkylalkyl are prepared by dehydrohalogenating compounds of the formula 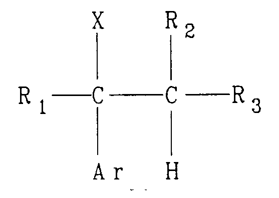
where X is a halogen atom, and Ar, R₁, R₂ and R₃ are as previously defined, in the presence of a catalyst based on palladium(0) combined with a ligand and an organic or inorganic base.

In the present specification, alkyl means straight or branched chain alkyl having 1 to 20 carbon atoms and includes, for example, methyl, ethyl, propyl, isopropyl, butyl, isobutyl, secondary butyl, tertiary butyl, pentyl, isopentyl, neopentyl, hexyl, heptyl, octyl, 2-ethylhexyl, 1,1,3,3-tetramethylbutyl, nonyl, decyl, dodecyl, tetradecyl, hexadecyl, octadecyl and eicosyl.

Cycloalkyl means cyclic alkyl having 3 to 7 carbon atoms and includes cyclopropyl, cyclobutyl, cyclopentyl, cyclohexyl and cycloheptyl.

Substituted phenyl and substituted naphthyl means phenyl or naphthyl substituted by at least one substituent selected from the group consisting of halogen (chlorine, bromine, fluorine or iodine), amino, nitro, hydroxy, alkyl, alkoxy which means straight or branched chain alkoxy having 1 to 10 carbon atoms, and includes, for example, methoxy, ethoxy, propoxy, isopropoxy, butoxy, isobutoxy, secondary butoxy, tertiary butoxy, pentyloxy, isopentyloxy, hexyloxy, heptyloxy, octyloxy, nonyloxy and decyloxy, phenoxy either unsubstituted or substituted with halogen, haloalkyl which means straight or alkyl having 1 to 8 carbon atoms which is substituted by at least one halogen, and includes, for example, chloromethyl, bromomethyl, fluoromethyl, iodomethyl, 2-chloroethyl, 2-bromoethyl, 2-fluoroethyl, 3-chloropropyl, 3-bromopropyl, 3-fluoropropyl, 4-chlorobutyl, 4-fluorobutyl, dichloromethyl, dibromomethyl, difluoromethyl, diiodomethyl, 2,2-dichloroethyl, 2,2-dibromoethyl, 2,2-difluoroethyl, 3,3-dichloropropyl, 3,3-difluoropropyl, 4,4-dichlorobutyl, 4,4-difluorobutyl, trichloromethyl, trifluoromethyl, 2,2,2-trifluoroethyl, 2,3,3-trifiuoropropyl, 1,1,2,2-tetrafluoroethyl and 2,2,3,3-tetrafluoropropyl.

Heteroaryl means 5 to 10 membered mono- or fused- heteroaromatic ring which has at least one heteroatom selected from the group consisting of nitrogen, oxygen and sulfur, and includes, for example, 2-furyl, 3-furyl, 2-thienyl, 3-thienyl, 2-pyridyl, 3-pyridyl, 4-pyridyl, pyrazolyl, imidazolyl, pyrimidinyl, pyridazinyl, pyrazinyl, benzimidazolyl, quinolyl, oxazolyl, thiazolyl and indolyl.

Substituted heteroaryl means 5 to 10 membered mono- or fused-heteroaromatic ring which has at least one heteroatom selected from the group consisting of nitrogen, oxygen and sulfur and which is substituted by at least one substituent selected from the group consisting of halogen, amino, nitro, hydroxy, alkyl, alkoxy and haloalkyl on the above-mentioned heteroaromatic nucleus.

Alkoxyalkyl means that the alkoxy moiety and the alkyl moiety each are straight or branched chain having 1 to 8 carbon atoms, and includes, for example, methoxymethyl, ethoxymethyl, propoxymethyl, isopropoxymethyl, butoxymethyl, isobutoxymethyl, tertiary butoxymethyl, pentyloxymethyl, hexyloxymethyl, heptyloxymethyl, octyloxymethyl, 2-methoxyethyl, 2-ethoxyethyl, 2-propoxyethyl, 2-butoxyethyl, 2-hexyloxyethyl, 2-octyloxyethyl, 3-methoxypropyl, 3-ethoxypropyl, 3-propoxypropyl, 3-butoxypropyl, 3-hexyloxypropyl, 3-octyloxypropyl, 4-methoxybutyl, 4-ethoxybutyl, 4-propoxybutyl, 4-butoxybutyl, 4-hexyloxybutyl, 4-octyloxybutyl, 5-methoxypentyl, 5-ethoxypentyl, 5-propoxypentyl, 5-butoxypentyl, 5-pentyloxypentyl, 5-hexyloxypentyl, 5-octyloxypentyl, 6-methoxyhexyl, 6-ethoxyhexyl, 6-propoxyhexyl, 6-butoxyhexyl, 6-pentyloxyhexyl, 6-hexyloxyhexyl, 6-octyloxyhexyl, 8-methoxyoctyl, 8-ethoxyoctyl, 8-butoxyoctyl, 8-hexyloxyoctyl and 8-octyloxyoctyl.

Alkylthioalkyl means that the alkylthio moiety and the alkyl moiety each are straight or branched chain ones having 1 to 8 carbon atoms and includes, for example, methylthiomethyl, ethylthiomethyl, propylthiomethyl, isopropylthiomethyl, butylthiomethyl, isobutylthiomethyl, tertiary butylthiomethyl, pentylthiomethyl, hexylthiomethyl, octylthiomethyl, 2-methylthioethyl, 2-ethylthioethyl, 2-butylthioethyl, 2-hexylthioethyl, 3-methylthiopropyl, 3-ethylthiopropyl, 3-propylthiopropyl, 3-butylthiopropyl, 4-methylthiobutyl, 4-ethylthiobutyl, 4-propylthiobutyl, 4-butylthiobutyl, 6-methylthiohexyl, 6-ethylthiohexyl, 6-butylthiohexyl, 8-methylthiooctyl, 8-ethylthiooctyl and 8-butylthiooctyl.

Phenylalkyl means that the alkyl moiety is straight or branched chain alkyl having 1 to 8 carbon atoms and includes, for example, benzyl, 2-phenylethyl, 1-phenylethyl, 3-phenylpropyl, 4-phenylbutyl, 5-phenylpentyl, 6-phenylhexyl and 8-phenyloctyl.

Substituted phenylalkyl means above-mentioned phenylalkyl which is substituted by at least one substituent at position 2, 3 or 4 selected from the group consisting of halogen, amino, nitro, hydroxy, alkyl, alkoxy and haloalkyl on the phenyl nucleus.

Heteroarylalkyl means that the heteroaryl moiety is 5 to 10 member monoor fused-heteroaromatic ring having at least one heteroatom selected from the group consisting of nitrogen, oxygen and sulfur as mentioned above and the alkyl moiety is straight or branched chain alkyl having 1 to 8 carbon atoms, and includes, for example, furfuryl, 3-furylmethyl, 2-thenyl, 3-thenyl, 2-, 3- or 4-pyridylmethyl, pyrazolylmethyl, 1-imidazolylmethyl, pyrimidinylmethyl, benzmidazolylmethyl, 2-(2-furyl)ethyl, 2-(2-thienyl)ethyl, 2-(2-pyridyl)ethyl, 2-(1-imidazolyl)ethyl, 3-(2-furyl)propyl, 3-(2-thienyl)propyl, 3-(2-pyridyl)propyl, 4-(2-furyl)butyl, 4-(2-thienyl)butyl and 4-(2-pyridyl)butyl.

Substituted heteroarylalkyl means that the substituted heteroaryl moiety is 5 to 10 membered mono- or fused-heteroaromatic ring which is substituted by at least one substituent selected from the group consisting of halogen, amino,nitro, hydroxy, alkyl, alkoxy and haloalkyl on the heteroaryl nucleus and which has at least one heteroatom selected from the group consisting of nitrogen, oxygen and sulfur as mentioned above, and that the alkyl moiety is straight or branched chain alkyl having 1 to 8 carbon atoms.

Cycloalkylalkyl means that the cycloalkyl moiety is cyclic alkyl having 3 to 7 carbon atoms and the alkyl moiety is straight or branched chain alkyl having 1 to 8 carbon atoms, and includes, for example, cyclopropylmethyl, cyclobutylmethyl, cyclopentylmethyl, cyclohexylmethyl, cycloheptylmethyl, 2-cyclopropylethyl, 2-cyclopentylethyl, 2-cyclohexylethyl, 3-cyclopropylpropyl, 3-cyclopentylpropyl, 3-cyclohexylpropyl 4-cyclopropylbutyl, 4-cyclopentylbutyl, 4-cyclohexylbutyl, 6-cyclopropylhexyl and 6-cyclohexylhexyl.

The halocarbons employed as starting materials in the process of the present invention are halocarbon compounds having the formula 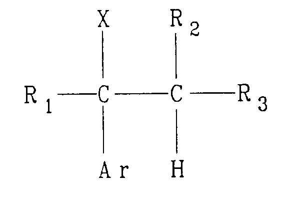
where X is a halogen atom, Ar is phenyl, substituted phenyl, naphthyl, substituted naphthyl, heteroaryl or substituted heteroaryl. R₁, R₂ and R₃ are individually the same or different and are hydrogen, alkyl, cycloalkyl, alkoxyalkyl, alkylthioalkyl, phenylalkyl, substituted phenylalkyl, heteroarylalkyl, substituted heteroarylalkyl or cycloalkylalkyl.

In the above halocarbons, it is preferred that Ar is phenyl; substituted phenyl, the substituents being alkyl of 1 to 12 linear or branched carbon atoms or halogen; phenoxy substituted with halogen; naphthyl or substituted naphthyl, the substituents being alkoxy. Most preferably, Ar is phenyl substituted with alkyl of 1 to 6 linear or branched carbon atoms or fluoro or naphthyl substituted with alkoxy having 1 to 6 linear or branched carbon atoms.

In the preferred and most preferred halocarbons described above, it is preferred that R₁, R₂ and R₃ are the same or different and are hydrogen, alkyl of 1 to 12 linear or branched carbon atoms, phenylalkyl, substituted phenylalkyl, the substituents being C₁ to C₆ linear or branched alkyl or halogen or heteroarylalkyl. Most preferably, R₁, R₂ and R₃ are different and are hydrogen, alkyl of 1 to 6 linear or branched carbon atoms, phenylalkyl, the alkyl group having from 1 to 4 linear or branched carbon atoms, or substituted phenylalkyl, the substituents being alkyl of 1 to 4 carbon atoms, or fluoro. Particularly preferred halocarbons are:
4-(1-chloroethyl)-isobutylbenzene
5-(1-chloroethyl)-methoxynapthalene
4-(1-bromoethyl)-isobutylbenzene
3-(1-chloroethyl)-benzoylbenzene
1-(1-chloroethyl)-3-fluoro-4-phenylbenzene
(1-bromoethyl)-benzene
(1-bromoethyl)-tribromobenzene
In order to effectively carry out the dehydrohalogenation process of the present invention, it is necessary to have an organic or inorganic base present to facilitate the removal of hydrogen chloride from the reaction medium. The organic base of use herein is typically a tertiary aryl, alkyl or mixed aryl/ alkyl amine such as trimethylamine, triphenylamine, or dimethylphenylamine. The inorganic base is generally an alkaline earth or alkali metal carbonate, bicarbonate or hydroxide. Preferably, the inorganic base is sodium or potassium carbonate. The bases are added to the reaction mixture typically as a finely ground powder. Typically, an equimolar quantity of base is employed based on the amount of halocarbon starting material.

The dehydrohalogenation process of this invention is conducted in the presence of a reaction-promoting quantity of (a) a palladium compound in which the palladium has a valence of 0 and (b) at least one acid-stable ligand. Ligands which may be used include monodentate or multidentate electron-donating substances such as those containing elements P, N, or O, and those containing multiple bonds such as olefinic compounds. Examples of such acid-stable ligands are trihydrocarbylphosphines, including trialkyl- and triarylphosphines, such as tri-n-butyl-, tricyclohexyl-, and triphenylphosphine; lower alkyl and aryl nitriles, such as benzonitrile and n-propionitrile; ligands containing pi-electrons, such as an allyl compound or 1,5-cyclooctadiene;piperidiene, piperazine, trichlorostannate(II), and acetylacetonate. In one embodiment, the palladium and ligand are added as a preformed complex of palladium, such as tetrakis(triphenylphosphine)palladium(0), or any other similar complex. In a preferred embodiment, active catalytic species are formed in situ by the addition to the reaction mixture of the individual components, i.e., a ligand and palladium. In the most preferred embodiment, triphenylphosphine and palladium are used and are added individually or together, either simultaneously or sequentially.

The amount of palladium preferably employed is such as to provide from 4 to 8000 moles of halocarbon per mole of palladium. More preferred is an amount to provide 100 to 4000 moles of halocarbon per mole of palladium. The most preferred amount provides 200 to 2000 moles of halocarbon per mole of palladium. The process of this invention is conducted in the presence of at least one mole of ligand per mole of palladium. More preferably 2 to 40 moles of ligand per mole of palladium are present, and most preferably 4 to 20 moles of ligand per mole of palladium are used. Even more highly preferred is an amount from 8 to 12 moles of ligand per mole of palladium.

The dehydrohalogenation of the halocarbon is conducted at a temperature between 10°C and 50°C, preferably 20-40°C, and most preferably 25-35°C. Higher temperatures can also be used. It has been found that a small advantage in yield is obtained by gradually increasing the temperature within the preferred ranges during the course of the reaction.

The reaction is typically carried out in an anhydrous organic solvent. Those solvents which can be used include one or more of the following: ketones, for example, acetone, methyl ethyl ketone, diethyl ketone, methyl-n-propyl ketone, and acetophenone; linear, poly and cyclic ethers, for example, diethyl ether, di-n-propyl ether, di-n-butyl ether, ethyl-n-propyl ether, glyme (the dimethyl ether of ethylene glycol), diglyme (the dimethyl ether of diethylene glycol), tetrahydrofuran, dioxane, and 1,3-dioxolane and aromatic hydrocarbons, for example, toluene, ethyl benzene and xylenes. Alcohols are also suitable as solvents, for example, methanol, ethanol, 1-propanol, 2-propanol, and isomers of butanol, isomers of pentanol. Acids and esters may also be used, such as formic or acetic acid or ethyl acetate. Most highly preferred are ketones, especially acetone and methyl ethyl ketone. The amount of solvent used can be up to 100 mL per gram of halocarbon, but the process is most advantageously conducted in the presence of 1 to 10 mL per gram of halocarbon.

The aryl-substituted olefins produced by the process of the present invention are useful intermediates as precursors to "profen-like" compounds, i.e., 2-aryl propionic acids, such as ibuprofen and naproxen.

Thus, carbonylation of 4-isobutylstyrene yields ibuprofen in a single step. Similarly, treatment of such styrene compound with HCN will produce a readily hydrolyzed nitrile intermediate.

### EXAMPLES

### Example 1

### Dehydrohalogenation of 4-(1-Chloroethyl)isobutylbenzene

A mixture of 0.11 g (0.56 mmol) of 4-(1-chloroethyl)isobutylbenzene (CEBB), 0.21 g (1.5 mmol) of anhydrous potassium carbonate, 6.0 mg (0.0052 mmol) of tetrakis(triphenylphosphine)palladium, and 10 mL of methyl ethyl ketone was heated at reflux for 5 hours. Analysis of the liquid phase by NMR indicated only isobutylstyrene (IBS) was present.

### Example 2

### Comparative

When the reaction of Example 1 was carried out at room temperature for 18 hours, NMR analysis indicated the presence of unreacted CEBB (43%) and IBS (57%).

### Example 3

### Comparative

When the reaction of Example 1 was carried out in the absence of potassium carbonate, only unreacted CEBB was observed.

### Example 4

### Comparative

When the reaction of Example 1 was carried out in the absence of tetrakis(triphenylphosphine)palladium, only unreacted CEBB was observed.

### Example 5

### Comparative

When the reaction of Example 1 was carried out using bis(triphenylphosphine)palladium chloride in place of tetrakis(triphenylphosphine)palladium, only unreacted CEBB was observed.

### Example 6

### Dehydrohalogenation of 4-(1-Bromoethyl)isobutylbenzene

A mixture of 0.12 g (0.50 mmol) of 4-(1-bromoethyl)-isobutylbenzene (BEBB), 0.21 g (1.5 mmol) of anhydrous potassium carbonate, 6.0 mg (0.0052 mmol) of tetrakis(triphenylphosphine)-palladium, and 10 mL of methyl ethyl ketone was heated at reflux for 3 hours. Analysis of the liquid phase by NMR indicated that unreacted BEBB (65%) and IBS (35%) were present.

### Example 7

### Dehydrohalogenation of (1-Bromoethyl)benzene

A mixture of 0.93 g (5.0 mmol) of (1-bromoethyl)benzene (BEB), 2.1 g (15 mmol) of anhydrous potassium carbonate, 60 mg (0.052 mmol) of tetrakis(triphenylphosphine)palladium, and 20 mL of methyl ethyl ketone was heated at 90-92°C for 6 hours. Analysis of the liquid phase by GC and NMR indicated that conversion was 94%, and that styrene and styrene oligomers were the products.

### Example 8

When the reaction of Example 7 was repeated using sodium hydroxide in place of potassium carbonate, conversion was 99% to styrene and styrene oligomers.

### Example 9

When the reaction of Example 7 was repeated using sodium hydroxide in place of potassium carbonate, toluene in place of methyl ethyl ketone, and at 100-110°C for 4 hours, conversion was 32% to styrene and styrene oligomers.

## Claims

1. Process for preparing compounds of the formula 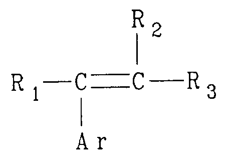 when Ar is phenyl, substituted phenyl, naphthyl, substituted naphthyl, heteroaryl or substituted heteroaryl; R₁, R₂ and R₃ are individually the same or different and are hydrogen, alkyl, cycloalkyl, alkoxyalkyl, alkylthioalkyl, phenylalkyl, substituted phenylalkyl, heteroarylalkyl, substituted heteroarylalkyl or cycloalkylalkyl, comprising dehydrohalogenating compounds of the formula 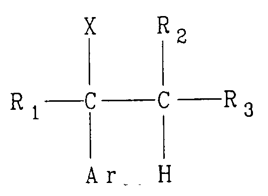 where X is a halogen atom, Ar, R₁, R₂ and R₃ are as previously defined in the presence of a catalyst based on palladium(0) combined with a ligand and an organic or inorganic base.

2. The process according to Claim 1 where Ar is phenyl, substituted phenyl, the substituents being C₁ to C₁₂ linear or branched alkyl, halogen, phenoxy substituted with halogen, naphthyl or naphthyl substituted with alkoxy.

3. The process according to Claim 2 wherein Ar is phenyl substituted with C₁ to C₆ linear or branched alkyl or fluoro or naphthyl substituted with C₁ to C₆ linear or branched alkoxy.

4. The process according to Claim 2, wherein R₁, R₂ and R₃ are the same or different and are hydrogen, C₁ to C₁₂ linear or branched alkyl, phenylalkyl, the phenyl group substituted with C₁ to C₆ linear or branched alkyl or halogen or heteroarylalkyl.

5. The process according to Claim 4 wherein R₁, R₂ and R₃ are different and are hydrogen, C₁ to C₆ linear or branched alkyl, phenylalkyl, the phenyl group substituted with C₁ to C₄ linear or branched alkyl or fluoro or phenylalkyl, the alkyl group having 1 to 4 linear or branched carbon atoms.

6. The process according to Claim 1 wherein said organic or inorganic base is an organic base that is a tertiary arylamine, tertiary alkylamine or a mixed tertiary aryl/alkyl-amine.

7. The process according to Claim 6 wherein said organic base is trimethylamine, triphenylamine or dimethylphenylamine.

8. The process according to Claim 1 wherein said organic or inorganic base is an inorganic base that is an alkaline earth or alkali metal carbonate, bicarbonate or hydroxide.

9. The process according to Claim 8 wherein said inorganic base is potassium or sodium carbonate.

10. A process for preparing 4-isobutylstyrene comprising dehydrochlorinating 4-(1-chloroethyl)isobutylbenzene in the presence of i) a catalytically effective amount of palladium (0) and a phosphorus-containing ligand and ii) an organic or inorganic base.

## Patentansprüche

1. Verfahren zur Herstellung von Verbindungen der Formel 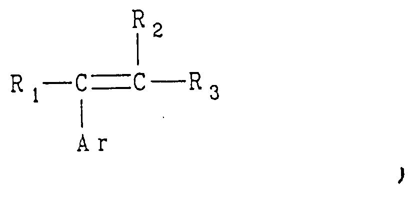 in der Ar Phenyl, substituiertes Phenyl, Naphthyl, substituiertes Naphthyl, Heteroaryl oder substituiertes Heteroaryl ist; R₁, R₂ und R₃ jeweils gleich oder verschieden sind und Wasserstoff, Alkyl, Cycloalkyl, Alkoxyalkyl, Alkylthioalkyl, Phenylalkyl, substituiertes Phenylalkyl, Heteroarylalkyl, substituiertes Heteroarylalkyl oder Cycloalkylalkyl bedeuten,
welches die Dehydrohalogenierung von Verbindungen der Formel 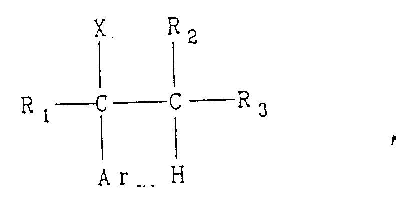 in der X ein Halogenatom ist, Ar, R₁, R₂ und R₃ die vorstehende Bedeutung haben, in Gegenwart eines Katalysators auf Palladium (0) Basis in Kombination mit einem Liganden und einer organischen oder anorganischen Base umfaßt.

2. Verfahren nach Anspruch 1, bei dem Ar Phenyl, substituiertes Phenyl, wobei die Substituenten lineares oder verzweigtes C₁-C₁₂Alkyl und Halogen darstellen, mit Halogen substituiertes Phenoxy sowie Naphthyl oder mit Alkoxy substituiertes Naphthyl ist.

3. Verfahren nach Anspruch 2, bei dem Ar mit linearem oder verzweigtem C₁-C₆Alkyl oder Fluor substituiertes Phenyl oder mit linearem oder verzweigtem Alkoxy substituiertes Naphthyl ist.

4. Verfahren nach Anspruch 2, bei dem R₁, R₂ und R₃ gleich oder verschieden sind und Wasserstoff, lineares oder verzweigtes C₁-C₁₂-Alkyl, Phenylalkyl, wobei die Phenylgruppe mit linearem oder verzweigtem C₁-C₆-Alkyl oder Halogen substituiert ist, oder Heteroarylalkyl bedeuten.

5. Verfahren nach Anspruch 4, bei dem R₁, R₂ und R₃ verschieden sind und Wasserstoff, lineares oder verzweigtes C₁-C₆-Alkyl, Phenylalkyl, wobei die Phenylgruppe mit linearem oder verzweigtem C₁-C₄-Alkyl oder Fluor substituiert ist, oder Phenylalkyl bedeuten, wobei die Alkylgruppe 1 bis 4 lineare oder verzweigte Kohlenstoffatome aufweist.

6. Verfahren nach Anspruch 1, bei dem die organische oder anorganische Base eine organische Base ist, bei der es sich um ein tertiäres Arylamin, tertiäres Alkylamin oder ein gemischtes tertiäres Aryl-/Alkylamin handelt.

7. Verfahren nach Anspruch 6, bei dem die organische Base Trimethylamin, Triphenylamin oder Dimethylphenylamin ist.

8. Verfahren nach Anspruch 1, bei dem die organische oder anorganische Base eine anorganische Base ist, bei der es sich um ein Erdalkali- oder Alkalimetallcarbonat, -bicarbonat oder -hydroxid handelt.

9. Verfahren nach Anspruch 8, bei dem die anorganische Base Kalium- oder Natriumcarbonat ist.

10. Verfahren zur Herstellung von 4-Isobutylstyrol, bei dem 4-(1-Chlorethyl)isobutylbenzol in Gegenwart von i) einer katalytisch wirksamen Menge von Palladium (0) und einem phosphorhaltigen Liganden und ii) einer organischen oder anorganischen Base dehydrochloriert wird.

## Revendications

1. Procédé de préparation de composés de formule 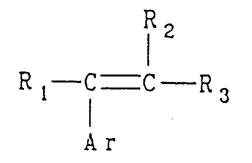 dans laquelle Ar représente un groupe phényle, phényle substitué, naphtyle, naphtyle substitué, hétéroaryle ou hétéroaryle substitue ; R₁, R₂ et R₃ sont individuellement identiques ou différents et représentent l'hydrogène, des groupes alkyle, cycloalkyle, alkoxyalkyle, alkylthioalkyle, phénylalkyle, phénylalkyle substitué, hétéroarylalkyle, hétéroarylalkyle substitué ou cycloalkylalkyle, comprenant la déshydrohalogénation de composés de formule 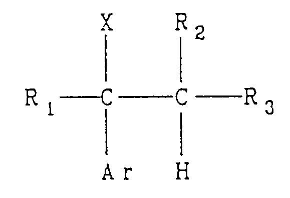 dans laquelle X représente un atome d'halogène, Ar, R₁, R₂ et R₃ répondent aux définitions précitées, en présence d'un catalyseur à base de palladium (0) associé à un ligand et une base organique ou inorganique.

2. Procédé suivant la revendication 1, dans lequel Ar représente un groupe phényle, phényle substitué, les substituants étant des groupes alkyle linéaires ou ramifiés en C₁ à C₁₂, halogéno, phénoxy substitué avec un halogène, naphtyle ou naphtyle substitué avec un groupe alkoxy.

3. Procédé suivant la revendication 2, dans lequel Ar représente un groupe phényle substitué avec un groupe alkyle linéaire ou ramifié en C₁ à C₆, fluoro ou naphtyle substitué avec un groupe alkoxy linéaire ou ramifié en C₁ à C₆.

4. Procédé suivant la revendication 2, dans lequel R₁, R₂ et R₃ sont identiques ou différents et représentent l'hydrogène, des groupes alkyle linéaires ou ramifiés en C₁ à C₁₂, phénylalkyle, le groupe phényle étant substitué avec un groupe alkyle linéaire ou ramifié en C₁ à C₆ ou halogéno, ou hétéroarylalkyle.

5. Procédé suivant la revendication 4, dans lequel R₁, R₂ et R₃ sont différents et représentent l'hydrogène, des groupes alkyle linéaires ou ramifiés en C₁ à C₆, phénylalkyle, le groupe phényle étant substitué avec un groupe alkyle linéaire ou ramifié en C₁ à C₄ ou fluoro, ou bien phénylalkyle, le groupe alkyle étant une chaîne linéaire ou ramifiée de 1 à 4 atomes de carbone.

6. Procédé suivant la revendication 1, dans lequel la base organique ou inorganique est une base organique qui est une arylamine tertiaire, une alkylamine tertiaire ou une aryl/alkylamine tertiaire mixte.

7. Procédé suivant la revendication 6, dans lequel la base organique est la triméthylamine, la triphénylamine ou la diméthylphénylamine.

8. Procédé suivant la revendication 1, dans lequel la base organique ou inorganique est une base inorganique qui est un carbonate, bicarbonate ou hydroxyde de métal alcalino-terreux ou de métal alcalin.

9. Procédé suivant la revendication 8, dans lequel la base inorganique est le carbonate de potassium ou de sodium.

10. Procédé de préparation de 4-isobutylstyrène, comprenant la déshydrochloration de 4-(1-chloréthyl)isobutylbenzène en présence i) d'une quantité catalytiquement efficace de palladium (0) et d'un ligand contenant du phosphore, et ii) d'une base organique ou inorganique.
